(19) Europäisches Patentamt
European Patent Office
Office européen des brevets

(11) **EP 1 481 677 B1**

(12) **FASCICULE DE BREVET EUROPEEN**

(45) Date de publication et mention
de la délivrance du brevet:
**19.03.2008 Bulletin 2008/12**

(21) Numéro de dépôt: **04291157.8**

(22) Date de dépôt: **05.05.2004**

(51) Int Cl.:
*A61K 31/375* (2006.01)    *A61K 8/67* (2006.01)
*A61K 8/72* (2006.01)    *A61K 47/34* (2006.01)
*A61K 47/30* (2006.01)    *A61P 17/00* (2006.01)
*A61Q 17/04* (2006.01)    *A61Q 19/00* (2006.01)
*A61Q 19/08* (2006.01)

(54) **Composition cosmétique et/ou dermatologique contenant l'acide ascorbique stabilisé par au moins un polymère amphiphile choisi parmi les oligomères ou polymères dérivés de polyisobutylène**

Kosmetische und/oder dermatologische Zusammensetzung enthaltend Ascorbinsäure stabilisiert durch mindestens ein amphiphiles Polymer ausgewählt aus Oligomeren oder Polymeren abgeleitet von Polyisobutylen

Cosmetic and/or dermatological composition containing ascorbic acid stabilised by at least one amphiphilic polymer chosen from oligomers or polymers derived from polyisobutylene

(84) Etats contractants désignés:
**AT BE BG CH CY CZ DE DK EE ES FI FR GB GR HU IE IT LI LU MC NL PL PT RO SE SI SK TR**

(30) Priorité: **27.05.2003 FR 0306421**

(43) Date de publication de la demande:
**01.12.2004 Bulletin 2004/49**

(73) Titulaire: **L'ORÉAL**
**75008 Paris (FR)**

(72) Inventeurs:
• **Biatry, Bruno**
**94300 Vincennes (FR)**
• **Simonnet, Jean-Thierry**
**94240 Cachan (FR)**

(74) Mandataire: **Kromer, Christophe**
**L'OREAL - D.I.P.I.**
**25-29 Quai Aulagnier**
**92600 Asnières (FR)**

(56) Documents cités:
**EP-A- 1 302 190        EP-A- 1 302 197**
**EP-A- 1 316 303        WO-A-02/055039**
**US-A1- 2002 039 565        US-A1- 2003 036 490**
**US-A1- 2003 059 391**

**Description**

La présente invention se rapporte à une composition cosmétique et/ou dermatologique à usage topique comprenant de l'acide ascorbique et au moins un polymère amphiphile choisi parmi les oligomères ou polymères dérivés de polyisobutylène.

Il est connu d'introduire dans des compositions cosmétiques divers actifs destinés à apporter des traitements spécifiques à la peau et/ou aux cheveux. Toutefois, certains de ces actifs présentent l'inconvénient d'être instables en milieu aqueux et de se dégrader facilement au contact de l'eau, en particulier à cause de phénomènes d'oxydation. Ils perdent ainsi rapidement leur activité au cours du temps et cette instabilité va à l'encontre de l'efficacité recherchée.

On cherche ainsi depuis longtemps à formuler l'acide ascorbique ou vitamine C, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau et possède une fonction anti-radicaux libres. Ces deux dernières propriétés en font un excellent candidat comme actif cosmétique ou dermatologique pour lutter contre le vieillissement de la peau ou prévenir celui-ci. L'acide ascorbique possède par ailleurs une activité dépigmentante sur la peau par blocage de la mélanogénèse par réduction de la DOPA quinone.

Malheureusement, en raison de sa structure chimique (d'alpha-cétolactone), l'acide ascorbique est très sensible à certains paramètres de l'environnement et notamment aux phénomènes d'oxydation. Il s'ensuit donc une dégradation rapide de l'acide ascorbique formulé en présence de ces paramètres, et plus particulièrement en présence d'oxygène, de lumière, d'ions métalliques, en fonction de la température, ou encore dans certaines conditions de pH (Pharm. Acta. Helv., 1969, 44, 611-667 ; STP Pharma, 1985, 4, 281-286).

Plusieurs solutions ont donc été envisagées dans l'art antérieur pour diminuer et/ou retarder la dégradation de l'acide ascorbique.

Il a ainsi été proposé d'utiliser l'acide ascorbique sous forme de dérivé chimique (ascorbyl phosphate de magnésium, ou esters d'acides gras et d'acide ascorbique), mais la biodisponibilité de ces dérivés est très faible (J. Am. Acad. Dermatol., 1996, 34, 29-33).

L'instabilité de l'acide ascorbique vis à vis de l'oxygène a pu être améliorée en utilisant des conditionnements particuliers comme des bi-compartiments sous atmosphère inerte tels que décrits dans le brevet US-5,935,584, ou bien encore par l'utilisation d'émulsions à deux phases dont l'une est constituée d'une poudre sèche contenant l'acide ascorbique et la seconde d'une phase liquide. Le mélange des deux phases doit s'effectuer au moment de l'utilisation (WO98/43598). Ces solutions présentent des inconvénients au niveau du coût et de la complexité des fabrications ainsi que des contraintes importantes au niveau de l'utilisation.

Une autre solution proposée dans l'art antérieur consiste à utiliser des glycols ou des polyols en forte concentration afin de diminuer la solubilité de l'oxygène dans la formulation, protégeant ainsi l'acide ascorbique (WO-96/24325, EP 0 755 674, US-5,981,578). Les polyols peuvent éventuellement être incorporés dans des liposomes comme décrit dans le brevet US-6,020,367. Mais ces solutions présentent l'inconvénient de conduire à des formulations collantes dont la cosméticité est difficile à améliorer. Par ailleurs la présence d'une forte concentration de ces composés peut provoquer des phénomènes d'irritation. La solubilité de l'acide ascorbique dans ces polyols étant insuffisante, il est parfois nécessaire d'utiliser des solubilisants tels que les N-alkylpyrrolidones (WO-00/78283) ou des dérivés de l'huile d'avocat (US-5,981,578), ce qui rend le procédé de formulation plus complexe.

L'acide ascorbique peut également être formulé dans des milieux anhydres tels que les silicones (US-6,194,452) qui sont capables de créer une barrière anhydre autour de l'acide ascorbique. Un inconvénient majeur de telles solutions résulte du manque de fraîcheur à l'application.

Il subsiste donc le besoin d'une composition utilisable notamment dans le domaine cosmétique, dans laquelle un actif hydrophile et instable en milieu oxydant est stabilisé, qui soit confortable lors de l'application, qui ne provoque aucune irritation de la peau après application, et qui soit compatible avec les contraintes d'une mise en oeuvre industrielle de son procédé de fabrication.

Le but de la présente invention est de proposer une composition comprenant l'acide ascorbique présentant de bonnes propriétés cosmétiques, tant au niveau du toucher qu'au niveau de la tolérance et dont la conservation dans le temps ne demande pas de précautions particulières.

La Demanderesse a découvert, de manière fortuite, que l'utilisation de certains polymères amphiphile choisi parmi les oligomères ou polymères dérivés de polyisobutylène comportant une partie apolaire polyisobutylène et au moins une partie polaire terminale dans des compositions dont la phase aqueuse renferme l'acide ascorbique, permettait d'atteindre le but précité.

Les oligomères et polymères dérivés de polyisobutylène sont connus dans d'autres domaines. Ainsi, ils sont décrits comme stabilisants d'émulsions explosives qui sont des émulsions inverses de nitrate d'ammonium fondu ou de solution saturée de nitrate d'ammonium dans une huile hydrocarbonée. Par ailleurs, ces composés sont connus comme stabilisants de compositions fertilisantes sous forme d'émulsion eau-dans-huile (voir le document US-A-5,518,517) en vue d'obtenir un relargage contrôlé des substances fertilisantes. En outre, il a été décrit dans la demande EP-1 172 089 des compositions, notamment cosmétiques, sous forme d'émulsions E/H contenant les composés précités. Ces composés sont stables et fraîches à l'application.

Dans les émulsions E/H décrites dans ces documents de l'art antérieur, la phase interne aqueuse contient des substances propres au domaine d'application de la composition, solubilisées ou dispersées dans l'eau. Il n'est toutefois pas fait mention de l'acide ascorbique et de ses dérivés. En outre, il n'est pas suggéré dans ces documents que les oligomères et polymères dérivés de polyisobutylène aient un quelconque pouvoir stabilisant vis-à-vis d'actifs hydrophiles sensibles à l'oxydation tels que l'acide ascorbique et ses dérivés.

La présente invention a donc pour objet une composition à usage topique comprenant, dans un milieu physiologiquement acceptable, de l'acide ascorbique et au moins un polymère amphiphile choisi parmi les oligomères ou polymères dérivés de polyisobutylène comportant une partie apolaire polyisobutylène comprenant au moins 40 atomes de carbone et au moins une partie polaire terminale constituée d'acides ou de diacides carboxyliques, de leurs anhydrides ou de leurs formes modifiées sous forme d'esters, d'amides ou de sels, et leurs mélanges, ladite composition étant dépourvue de polymère ou copolymère de N-vinylinimidazole. Ce dernier polymère est en effet déjà connu dans des compositions cosmétiques et/ou dermatologiques comprenant l'acide ascorbique et un polymère amphiphile dérivé de polyisobutylène (voir EP-A-1 316 303). Le polymère amphiphile est présent en quantité suffisante pour stabiliser ledit actif hydrophile vis-à-vis de l'oxydation.

Par polymère ou copolymère de N-vinylimidazole on entend tout polymère ou copolymère comportant une ou plusieurs unités N-vinylimidazole.

L'utilisation des polymères amphiphiles choisi parmi les oligomères ou polymères dérivés de polyisobutylène comportant une partie apolaire polyisobutylène comprenant au moins 40 atomes de carbone et au moins une partie polaire terminale constituée d'acides ou de diacides carboxyliques, de leurs anhydrides ou de leurs formes modifiées sous forme d'esters, d'amides ou de sels, et leurs mélanges, pour stabiliser l'acide ascorbique présente l'avantage de pouvoir stabiliser cet actif dans des compositions possédant un pH compris entre 5 et 7, c'est à dire dans des conditions qui respectent l'équilibre physiologique de la peau dont le pH est voisin de 5,5.

L'utilisation des polymères amphiphiles choisi parmi les oligomères ou polymères dérivés de polyisobutylène selon l'invention pour stabiliser l'acide ascorbique est particulièrement adaptée pour stabiliser ledit actif à une concentration comprise entre 0,5 et 20 % en poids, préférentiellement entre 1 et 15 % en poids par rapport au poids total de la composition.

Les polymères amphiphiles choisis parmi les oligomères et polymères utilisés comme émulsionnants dans la composition de l'invention sont constitués d'une partie apolaire polyisobutylène et d'au moins une partie polaire.

La partie apolaire polyisobutylène comprend au moins 40 atomes de carbone et de préférence de 60 à 700 atomes de carbone. Il est important que cette partie comporte au moins 40 atomes de carbone pour atteindre le but de l'invention. S'il y a moins de 40 atomes de carbone, on n'obtient pas un système bien stable.

La partie polaire des émulsionnants oligomères ou polymères de l'invention est constituée d'acides ou de diacides carboxyliques, de leurs anhydrides ou de leurs formes modifiées sous forme d'esters, d'amides ou de sels, et leurs mélanges. De préférence, la partie polaire terminale est constituée de diacides carboxyliques ou de leurs anhydrides ou de leurs formes modifiées sous forme d'esters, d'amides ou de sels.

Par formes modifiées sous forme d'esters, d'amides ou de sels, on désigne les acides ou diacides carboxyliques modifiés par des alcools, des amines, des alcanolamines ou des polyols, ou encore sous forme de sels de métal alcalin ou alcalino-terreux, d'ammonium ou encore de base organique comme les sels de diéthanolamine et de triéthanolamine.

Des émulsionnants oligomères ou polymères à partie polaire acide carboxylique peuvent être par exemple issus de la réaction entre un polyisobutylène et au moins un acide ou anhydride carboxylique choisi dans le groupe comprenant l'acide maléique, l'anhydride maléique, l'acide fumarique, l'acide itaconique, l'acide citraconique, l'acide mésaconique, l'acide aconitique, leurs formes modifiées sous forme d'esters, d'amides ou de sels, et leurs mélanges. De préférence, la partie polaire est constituée par l'acide ou l'anhydride succinique, leurs formes modifiées esters ou amides, les sels d'ions alcalins, alcalino-terreux ou organiques correspondants, ces composés pouvant éventuellement être polyoxyéthylénés.

Les émulsionnants dérivés d'acide ou d'anhydride succinique peuvent être choisis notamment parmi les dérivés polyisobutylène d'acide ou d'anhydride succinique décrits dans les brevets US-A-4,234,435, US-A-4,708,753, US-A-5,129,972, US-A-4,931,110, GB-A-2,156,799 et US-A-4,919,179. La partie polyisobutylène peut être hydrogénée ou non, de poids moléculaire allant de 400 à 5000. Dans le polyisobutylène à terminaison succinique ainsi obtenu, la partie succinique peut être estérifiée, amidifiée ou sous forme de sel, c'est-à-dire qu'elle peut être avantageusement modifiée par des alcools, des amines, des alcanolamines ou des polyols, ou encore se trouver sous forme de sels de métal alcalin ou alcalino-terreux, d'ammonium ou encore de base organique comme les sels de diéthanolamine et de triéthanolamine. Les polyisobutylènes à terminaison succinique estérifiée ou amidifiée sont des produits de réaction de (a) un polyisobutylène à terminaison succinique, et de (b) une amine ou un alcool, pour former une amide ou un ester. Le terme « amine » utilisé ici comprend tous types d'amines dont les alcanolamines. Il peut s'agir par exemple de monoamines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatique, cycloaliphatiques, aromatiques et hétérocycliques. Les polyisobutylènes à terminaison succinique modifiée (estérifiée ou amidifiée) et leur procédé de préparation sont décrits en particulier dans le document US-A-4,708,753.

Comme polyisobutylène à terminaison succinique, on peut citer notamment les polyisobutylènes à terminaison succinique modifiée, tels que les produits commercialisés sous les dénominations LUBRIZOL 5603 et LUBRIZOL 2650 par la société Lubrizol. Selon un mode préféré de réalisation de l'invention, on utilise le polymère commercialisé sous la dénomination LUBRIZOL 5603 par la société Lubrizol, qui est le sel de diéthyléthanolamine de polyisobutylène à terminaison succinique estérifiée (nom INCI : Hydroxyethyldiethonium polyisobutenyl triethylaminosuccinate / diethylethanolamine).

Un autre exemple de dérivé de polyisobutylène utilisable dans l'invention est le produit de la réaction de l'anhydride maléique avec le polyisobutylène, tel que le produit commercialisé sous la dénomination Glissopal SA par la société BASF.

La quantité de phase aqueuse dans les compositions selon l'invention est de préférence d'au moins 40 % en poids par rapport au poids total de la composition. Elle peut aller par exemple de 40 à 95 % en poids de préférence de 50 à 90 %, mieux de 60 à 90 % en poids et encore mieux de 80 à 90 % en poids par rapport au poids total de la composition. La composition de l'invention contient de préférence au moins 30 % en poids d'eau et mieux au moins 50 % en poids d'eau, par rapport au poids total de la composition.

La quantité d'oligomère(s) ou de polymère(s) dérivé(s) de polyisobutylène en matière active dans la composition de l'invention peut aller par exemple de 0,1 à 10 % en poids de matière active, de préférence de 0,5 à 5 % en poids et mieux de 1 à 3 % en poids par rapport au poids total de la composition. On peut utiliser un ou plusieurs oligomères ou polymères dérivés de polyisobutylène.

Dans les compositions selon la présente invention, le rapport molaire entre l'oligomère ou polymère dérivé de polyisobutylène et l'acide ascorbique varie entre 0,0006 et 3, de préférence entre 0,001 et 0,1, et plus particulièrement entre 0,01 et 0,5.

Selon un mode préféré de réalisation de l'invention, les oligomères ou polymères dérivés de polyisobutylène sont les seuls émulsionnants utilisés dans la composition selon l'invention.

Toutefois, on peut éventuellement ajouter d'autres agents amphiphiles habituellement utilisés, en particulier dans les émulsions eau-dans-huile, tels que les tensioactifs classiques ioniques, non ioniques, amphotères, zwitterioniques, les oligomères ou les polymères amphiphiles, les particules organiques ou inorganiques amphiphiles.

Les compositions utilisées selon l'invention sont destinées à une application topique sur la peau et/ou ses phanères et contiennent donc un milieu physiologiquement acceptable, c'est-à-dire compatible avec les tissus cutanés tels que la peau, le cuir chevelu, les cils, les sourcils, les cheveux, les ongles et les muqueuses. Ce milieu physiologiquement acceptable peut être plus particulièrement constitué d'eau et éventuellement d'un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol ; les polyéthylène glycols ayant de 6 à 80 unités oxyde d'éthylène ; les polyols comme le propylène glycol, l'isoprène glycol, le butylène glycol, la glycérine, le sorbitol.

Ce milieu physiologiquement acceptable est de préférence un milieu cosmétiquement acceptable, c'est-à-dire qu'il présente un aspect, une odeur et une couleur agréables et ne génère pas d'inconforts (picotements, tiraillements, rougeurs) inacceptables pour l'utilisateur. Quand le milieu physiologiquement acceptable est un milieu aqueux, il a ainsi généralement un pH compatible avec la peau, allant de préférence de 3 à 9 et mieux de 3,5 à 7,5.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique (liposomes, niosomes, oléosomes). Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Dans un aspect avantageux de l'invention, les compositions se présentent sous la forme d'émulsions eau dans huile.

Dans le cas des émulsions eau-dans-huile, la proportion de la phase aqueuse dans l'émulsion est de préférence d'au moins 40 % en poids par rapport au poids total de la composition. Elle peut être notamment comprise entre 40 et 95 % en poids, de préférence entre 50 et 90 % en poids, plus particulièrement entre 60 à 90 % en poids, et tout particulièrement entre 80 et 90 % en poids par rapport au poids total de la composition.

Les émulsions peuvent également contenir au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90$^R$ par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu. Il est également possible d'utiliser comme co-émulsionnant le tétrapropenyl disuccinate disodique vendu sous la dénomination Rewocoros B3010® par la société Witco.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants hydrophiles ou lipophiles, les conservateurs, les solvants, les parfums, les charges, les filtres UV, les bactéricides, les absorbeurs d'odeur, les matières colorantes, les extraits végétaux, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Selon un mode préféré de réalisation de l'invention, la composition utilisée selon l'invention contient au moins un filtre UV (ou filtre solaire) qui peut être un filtre chimique ou un filtre physique ou un mélange de tels filtres.

A titre d'illustration et de façon non limitative, on peut citer les familles suivantes (les noms correspondent à la nomenclature CTFA des filtres) :

les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 disponible chez B.A.S.F.) ; les benzylidènes-camphres, en particulier le 3-benzylidène-camphre, l'acide benzylidènecamphosulfonique, le benzalkoniumméthosulfate de Camphre, le polyacrylamidométhylbenzylidène camphre, l'acide téréphthalylidène di-camphre sulfonique , et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane, l'oxyde de zinc et le dioxyde de titane.

La quantité de filtres dépend de l'utilisation finale souhaitée. Elle peut aller par exemple de 1 à 20% en poids et mieux de 2 à 10% en poids par rapport au poids total de la composition.

Selon un autre mode de réalisation de l'invention, la composition utilisée contient en outre au moins un actif choisi parmi les agents dépigmentants, les inhibiteurs de NO-synthase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents tenseurs et les agents anti-pollution ou anti-radicalaires.

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; et les extraits de plantes, en particulier de réglisse, de mûrier et de scutellaire, sans que cette liste soit limitative.

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

Parmi les actifs stimulant les macromolécules du derme ou empêchant leur dégradation, on peut citer ceux qui agissent :

- soit sur la synthèse du collagène, tels que les extraits de Centella asiatica ; les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines.

- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;

- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;

- soit sur la synthèse de la fibronectine, tels que l'extrait de zooplancton Salina commercialisé par la société SEPORGA sous la dénomination commerciale GP4G® ;
l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;

- soit sur l'inhibition des métalloprotéinases (MMP) telles que plus particulièrement les MMP 1, 2, 3, 9 . On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;

- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parclastyl® ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl®.

Les agents myorelaxants utilisables dans la composition selon l'invention comprennent les inhibiteurs calciques tels que l'alvérine et ses sels, le gluconate de manganèse, les ouvreurs de canaux chlore tel que le Diazepam, et les inhibiteurs de catécholamines et d'acétylcholine tels que l'hexapeptide argireline R commercialisé par la société LIPOTEC.

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :

(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,

(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,

(3) les protéines et hydrolysats de protéines végétales, en particulier de maïs, de seigle, de froment, de sarrasin, de sésame, d'épautre, de pois, de fève, de lentille, de soja et de lupin, (3) les silicates mixtes, notamment les phyllosilicates et en particulier les Laponites,

(4) les microparticules de cire, choisies par exemple parmi les cires de Carnauba, de Candelila ou d'Alfa,

(5) les particules colloïdales de charge inorganique ayant un diamètre moyen en nombre compris entre 0,1 et 100 nm, de préférence entre 3 et 30 nm, et choisies par exemple parmi : la silice, l'oxyde de cérium, l'oxyde de zirconium, l'alumine, le carbonate de calcium, le sulfate de baryum, le sulfate de calcium, l'oxyde de zinc et le dioxyde de titane.

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl® , le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect® .

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichomia crassipes) ; et la fraction hydrosoluble de maïs commercialisée par la société SOLABIA sous la dénomination commerciale Phytovityl® .

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

La composition selon l'invention peut être appliquée sur la peau, les poils, les cils, les cheveux, les ongles ou les lèvres, suivant l'usage auquel elle est destinée. Elle est de préférence adaptée à une application topique sur la peau. Cette composition peut ainsi être utilisée dans un procédé de traitement cosmétique de la peau, comprenant l'application de la composition selon l'invention sur la peau, par exemple en vue de la tonifier, de la régénérer, de lisser les ridules de la peau, et/ou pour lutter contre le vieillissement cutané, contre les méfaits des rayonnements UV et/ou pour renforcer les tissus cutanés contre les agressions de l'environnement.

En variante, la composition selon l'invention peut être utilisée pour la fabrication d'une préparation dermatologique, telle qu'une préparation destinée à dépigmenter la peau, les poils et/ou les cheveux.

Les exemples qui suivent servent à illustrer l'invention. Les composés sont, selon le cas, cités en noms chimiques ou en noms CTFA (International Cosmetic Ingredient Dictionary and Handbook).

**Exemple 1 : Emulsion Eau dans Huile :**

| Phase A : | Isohexadécane | 8 g |
|---|---|---|
| | Squalane | 3,7 g |
| | Polydiméthylsiloxane | 4,1 g |
| | Huile d'amandes d'abricots | 2,3 g |
| | Sel de diéthyléthanolamine de polyisobutylène à terminaison succinique estérifiée * | 1,9 g |
| Phase B : | Acide ascorbique | 2 g |
| | Hydroxyde de potassium à 50 % | 1,2 g |
| | Eau déminéralisée | 67,8 g |
| | Glycérol | 5 g |
| | Conservateur | 1 g |
| Phase C : | Poudre de Nylon-12 | 3 g |
| (*) commercialisé sous la référence Lubrizol 5603® par la société Lubrizol. | | |

Mode opératoire : la phase B est émulsionnée lentement dans la phase A à température ambiante. La phase C est ensuite ajoutée.

### Exemple 1 Comparatif : Emulsion Eau dans huile :

| | | |
|---|---|---|
| Phase A : | Cyclopentasiloxane et | |
| | PEG/PPG-18/18 diméthicone * | 20 g |
| | Phényl triméthicone | 7 g |
| | Huile d'amandes d'abricots | 5,5 g |
| | Poudre de Nylon-12 | 3 g |
| Phase B : | Acide ascorbique | 2 g |
| | Hydroxyde de potassium à 50 % | 1,2 g |
| | Eau déminéralisée | 55,3 g |
| | Glycérol | 5 g |
| | Conservateur | 1 g |

*(\*) commercialisé sous la référence DC-2 5225 C par la société Dow Corning.*

Mode opératoire : à température ambiante, la phase B est émulsionnée lentement dans la phase A.

### Exemple 2 : Test de conservation accéléré :

Ce test a pour but d'étudier la dégradation de l'acide ascorbique dans une composition après deux mois de conservation à 45°C.

Le taux de dégradation mesuré est donné par le rapport :

$$(C_0 - C_{2mois})/C_0$$

avec $C_0$ concentration en acide ascorbique à t=0 et $C_{2mois}$ la concentration en acide ascorbique à t=2 mois.

La concentration en acide ascorbique est déterminée par la technique HPLC (système LaChrom Merck). Les conditions analytiques sont les suivantes:

Colonne Lichrosphere100 RP18 (250 mm)
Eluant : tampon phosphate O,1 M, pH 2,1
Débit : 1 ml/min.
Détection à 257 nm
Dilution de l'échantillon tel que la concentration en acide ascorbique soit comprise entre 0,05 et 1 mg/ml.

Le test a été réalisé avec la composition de l'exemple 1, et avec la composition de l'exemple comparatif 1 qui contient à la place du polymère selon l'invention, un émulsionnant classiquement utilisé, le Cyclopentasiloxane et PEG/PPG-18/18 diméthicone.

Les résultats obtenus sont regroupés dans le tableau suivant :

| | Taux de dégradation après 2 mois à 45°C (en %) | |
|---|---|---|
| | sous air, flacon verre ambré | sous azote, flacon aluminium |
| **Exemple 1** | 7,8 | 3,4 |
| **Exemple 1 comparatif** | 14,4 | 9,8 |

Les résultats ci-dessus montrent que la stabilité de l'acide ascorbique lorsqu'il est formulé en présence de sel de diéthyléthanolamine de polyisobutylène à terminaison succinique estérifiée (Lubrizol 5603) est augmentée de 50 %, même en présence d'oxygène.

**Exemple 3 : Emulsion Eau dans Huile :**

| | | | |
|---|---|---|---|
| Phase A : | Isononanoate d'isononyle | 4,65 g | |
| | Perhydrosqualène végétal | 3,48 g | |
| | Polydiméthylsiloxane (viscosité 10 est) | 2,33 g | |
| | Sel de diéthyléthanolamine de polyisobutylène à terminaison succinique estérifiée * | 1,92 g | |
| Phase B : | Acide ascorbique | 5 g | |
| | Hydroxyde de potassium à 50 % | 3,17 g | |
| | Eau déminéralisée | 67,95 g | |
| | Glycérol | 5 g | |
| | Conservateurs | 1 g | |
| | Ammonium polyacryloyldiméthyl taurate | 0,2 g | |
| | Sulfate de magnésium, 7H2O | 2 g | |
| Phase C : | Microsphères de silice | 3 g | |
| Phase D : | Parfum | 0,3 g | |

*(\*) commercialisé sous la référence Lubrizol 5603® par la société Lubizol.*

Mode opératoire : à température ambiante, la phase B est émulsionnée dans la phase A puis on introduit successivement les phases C et D. On obtient une crème blanche, douce et fraîche à l'application qui tonifie la peau et dans laquelle l'acide ascorbique présente une bonne stabilité.

**Revendications**

1. Composition à usage topique comprenant, dans un milieu physiologiquement acceptable, de l'acide ascorbique et au moins un polymère amphiphile choisi parmi les oligomères ou polymères dérivés de polyisobutylène comportant une partie apolaire polyisobutylène comprenant au moins 40 atomes de carbone et au moins une partie polaire terminale constituée d'acides ou de diacides carboxyliques, de leurs anhydrides ou de leurs formes modifiées sous forme d'esters, d'amides ou de sels, et leurs mélanges, ladite composition étant dépourvue de polymère ou copolymère de N-vinylimidazole.

2. Composition selon la revendication 1, **caractérisée en ce que** l'acide ascorbique est présent en une quantité comprise entre 0,5 % et 20 % en poids par rapport au poids total de la composition.

3. Composition selon l'une des revendications 1 ou 2, **caractérisée en ce que** la partie apolaire polyisobutylène du polymère amphiphile comprend de 60 à 700 atomes de carbone.

4. Composition selon l'une quelconque des revendications 1 à 3, **caractérisée en ce que** la partie polaire terminale du polymère amphiphile est constituée de diacides carboxyliques ou de leurs anhydrides ou de leurs formes modifiées sous forme d'esters, d'amides ou de sels.

5. Composition selon l'une quelconque des revendications 1 à 4, **caractérisée en ce que** l'oligomère ou le polymère dérivé de polyisobutylène est issu de la réaction entre un dérivé de polyisobutylène et au moins un acide ou anhydride choisi dans le groupe comprenant l'acide succinique, l'anhydride succinique, l'acide maléique ; l'anhydride maléique ; l'acide fumarique ; l'acide itaconique ; l'acide citraconique ; l'acide mésaconique ; l'acide aconitique ; leurs formes modifiées sous forme d'esters, d'amides ou de sels, et leurs mélanges.

6. Composition selon l'une quelconque des revendications 1 à 5, **caractérisée en ce que** l'oligomère ou le polymère dérivé de polyisobutylène est un polyisobutylène à terminaison succinique modifiée.

7. Composition selon l'une quelconque des revendications 1 à 6, **caractérisée en ce que** l'oligomère ou le polymère dérivé de polyisobutylène est le produit de la réaction de l'anhydride maléique avec le polyisobutylène.

8. Composition selon l'une quelconque des revendications 1 à 7, **caractérisée en ce que** le polymère dérivé de polyisobutylène est le sel de diéthyléthanolamine de polyisobutylène à terminaison succinique estérifiée.

9. Composition selon l'une quelconque des revendications 1 à 8, **caractérisée en ce que** la quantité d'oligomère ou de polymère dérivé de polyisobutylène est comprise entre 0,1 et 10 % en poids par rapport au poids total de la composition.

10. Composition selon l'une quelconque des revendications 1 à 9, **caractérisée en ce qu'**elle se présente sous la forme d'une émulsion eau dans huile.

11. Composition selon l'une quelconque des revendications 1 à 10 **caractérisée en ce que** le rapport molaire entre l'oligomère ou polymère dérivé de polyisobutylène et l'acide ascorbique varie entre 0,0006 et 3.

12. Composition selon l'une des revendications 1 à 11, **caractérisée en ce que** le polymère amphiphile choisi parmi les oligomères ou polymères dérivés de polyisobutylène comportant une partie apolaire polyisobutylène comprenant au moins 40 atomes de carbone et au moins une partie polaire terminale constituée d'acides ou de diacides carboxyliques, de leurs anhydrides ou de leurs dérivés, et leurs mélanges, est le seul émulsionnant présent dans la composition.

13. Procédé de traitement cosmétique comprenant l'application sur la peau d'une composition selon l'une quelconque des revendications 1 à 12.

14. Procédé de traitement cosmétique destiné à tonifier, régénérer, et/ou lisser les ridules de la peau, et/ou pour lutter contre le vieillissement cutané et/ou pour renforcer les tissus cutanés contre les agressions de l'environnement, comprenant l'application sur la peau d'une composition selon l'une quelconque des revendications 1 à 12.

15. Utilisation d'une composition selon l'une quelconque des revendications 1 à 12 pour la fabrication d'une préparation dermatologique destinée à dépigmenter la peau, les poils et/ou les cheveux et/ou à lutter contre les méfaits des rayonnements UV.

16. Utilisation cosmétique d'une composition selon l'une des revendications 1 à 12 pour tonifier, régénérer, et/ou lisser les ridules de la peau, et/ou pour lutter contre le vieillissement cutané et/ou pour renforcer les tissus cutanés contre les agressions de l'environnement

**Claims**

1. Composition for topical use comprising, in a physiologically acceptable medium, ascorbic acid and at least one amphiphilic polymer chosen from polyisobutylene-derived oligomers or polymers comprising a polyisobutylene apolar part containing at least 40 carbon atoms and at least one terminal polar part consisting of carboxylic acids or dicarboxylic acids, of anhydrides thereof or of modified forms thereof in the form of esters, amides or salts, and mixtures thereof, said composition being devoid of N-vinylimidazole polymer or copolymer.

2. Composition according to Claim 1, **characterized in that** the ascorbic acid is present in an amount of between 0.5% and 20% by weight relative to the total weight of the composition.

3. Composition according to either of Claims 1 and 2, **characterized in that** the polyisobutylene apolar part of the amphiphilic polymer contains from 60 to 700 carbon atoms.

4. Composition according to any one of Claims 1 to 3, **characterized in that** the terminal polar part of the amphiphilic polymer is constituted of dicarboxylic acids or of anhydrides thereof or of modified forms thereof in the form of esters, amides or salts.

5. Composition according to any one of Claims 1 to 4, **characterized in that** the polyisobutylene-derived oligomer or polymer is derived from the reaction between a polyisobutylene derivative and at least one acid or anhydride chosen from the group comprising succinic acid; succinic anhydride; maleic acid; maleic anhydride; fumaric acid; itaconic acid; citraconic acid; mesaconic acid; aconitic acid; modified forms thereof in the form of esters, amides or salts, and mixtures thereof.

6. Composition according to any one of Claims 1 to 5, **characterized in that** the polyisobutylene-derived oligomer or polymer is a polyisobutylene comprising a modified succinic ending.

7. Composition according to any one of Claims 1 to 6, **characterized in that** the polyisobutylene-derived oligomer or polymer is the product of the reaction of maleic anhydride with polyisobutylene.

8. Composition according to any one of Claims 1 to 7, **characterized in that** the polyisobutylene-derived polymer is the diethylethanolamine salt of polyisobutylene comprising an esterified succinic ending.

9. Composition according to any one of Claims 1 to 8, **characterized in that** the amount of polyisobutylene-derived oligomer or polymer is between 0.1% and 10% by weight relative to the total weight of the composition.

10. Composition according to any one of Claims 1 to 9, **characterized in that** it is in the form of a water-in-oil emulsion.

11. Composition according to any one of Claims 1 to 10, **characterized in that** the molar ratio between the polyisobutylene-derived oligomer or polymer and the ascorbic acid ranges between 0.0006 and 3.

12. Composition according to one of Claims 1 to 11, **characterized in that** the amphiphilic polymer chosen from polyisobutylene-derived oligomers or polymers comprising a polyisobutylene apolar part containing at least 40 carbon atoms and at least one terminal polar part being constituted of carboxylic acids or of dicarboxylic acids, of anhydrides thereof or of derivatives thereof, and mixtures thereof, is the only emulsifier present in the composition.

13. Cosmetic treatment process comprising the application to the skin of a composition according to any one of Claims 1 to 12.

14. Cosmetic treatment process for tonifying, regenerating and/or smoothing out the fine lines of the skin, and/or for combating skin ageing, and/or for strengthening skin tissues against environmental attack, comprising the application to the skin of a composition according to any one of Claims 1 to 12.

15. Use of a composition according to any one of Claim 1 to 12, for the manufacture of a dermatological preparation for depigmenting the skin, body hair and/or head hair, and/or for combating the harmful effects of UV radiation.

16. Cosmetic use of a composition according to one of Claims 1 to 12, for tonifying, regenerating and/or smoothing out the fine lines of the skin, and/or for combating skin ageing, and/or for strengthening skin tissues against environmental attack.

**Patentansprüche**

1. Zusammensetzung für die topische Anwendung, die in einem physiologisch akzeptablen Medium Ascorbinsäure und mindestens ein amphiphiles Polymer enthält, das unter den von Polyisobutylen abgeleiteten Oligomeren oder Polymeren, die einen apolaren Polyisobutylenteil mit mindestens 40 Kohlenstoffatomen und mindestens einen endständigen polaren Teil aufweisen, der aus Carbonsäuren oder Dicarbonsäuren, ihren Anhydriden oder ihren in Form von Estern, Amiden oder Salzen modifizierten Formen besteht, und deren Gemischen ausgewählt ist, wobei die Zusammensetzung kein N-Vinylimidazol-Polymer oder N-Vinylimidazol-Copolymer enthält.

2. Zusammensetzung nach Anspruch 1, **dadurch gekennzeichnet, dass** die Ascorbinsäure in einer Menge von 0,5 bis 20 Gew.-%, bezogen auf das Gesamtgewicht der Zusammensetzung, enthalten ist.

3. Zusammensetzung nach einem der Ansprüche 1 oder 2, **dadurch gekennzeichnet, dass** der apolare Polyisobutylenteil des amphiphilen Polymers 60 bis 700 Kohlenstoffatome umfasst.

4. Zusammensetzung nach einem der Ansprüche 1 bis 3, **dadurch gekennzeichnet, dass** der endständige polare Teil des amphiphilen Polymers aus Dicarbonsäuren oder deren Anhydriden oder ihren in Form von Estern, Amiden oder Salzen modifizierten Formen besteht.

5. Zusammensetzung nach einem der Ansprüche 1 bis 4, **dadurch gekennzeichnet, dass** das von Polyisobutylen abgeleitete Oligomer oder Polymer aus der Reaktion eines Polyisobutylenderivats und mindestens einer Säure oder eines Anhydrids stammt, die unter Bernsteinsäure, Bernsteinsäureanhydrid, Maleinsäure; Maleinsäureanhydrid; Fumarsäure; Itaconsäure; Citraconsäure; Mesaconsäure; Aconitsäure; deren in Form von Estern, Amiden oder Salzen modifizierten Formen; und deren Gemischen ausgewählt sind.

6. Zusammensetzung nach einem der Ansprüche 1 bis 5, **dadurch gekennzeichnet, dass** das vom Polyisobutylen abgeleitete Oligomer oder Polymer ein Polyisobutylen mit modifizierter Bernsteinsäureendgruppe ist.

7. Zusammensetzung nach einem der Ansprüche 1 bis 6, **dadurch gekennzeichnet, dass** das von Polyisobutylen abgeleitete Oligomer oder Polymer das Reaktionsprodukt von Maleinsäureanhydrid und Polyisobutylen ist.

8. Zusammensetzung nach einem der Ansprüche 1 bis 7, **dadurch gekennzeichnet, dass** das von Polyisobutylen abgeleitete Polymer das Diethylethanolaminsalz von Polyisobutylen mit endständiger veresterter Bernsteinsäuregruppe ist.

9. Zusammensetzung nach einem der Ansprüche 1 bis 8, **dadurch gekennzeichnet, dass** der Mengenanteil des von Polyisobutylen abgeleiteten Oligomers oder Polymers im Bereich von 0,1 bis 10 Gew.-%, bezogen auf das Gesamtgewicht der Zusammensetzung, liegt.

10. Zusammensetzung nach einem der Ansprüche 1 bis 9, **dadurch gekennzeichnet, dass** sie als Wasser-in-Öl-Emulsion vorliegt.

11. Zusammensetzung nach einem der Ansprüche 1 bis 10, **dadurch gekennzeichnet, dass** das Molverhältnis des von Polyisobutylen abgeleiteten Oligomers oder Polymers und der Ascorbinsäure im Bereich von 0,0006 bis 3 liegt.

12. Zusammensetzung nach einem der Ansprüche 1 bis 11, **dadurch gekennzeichnet, dass** das amphiphile Polymer, das unter den von Polyisobutylen abgeleiteten Oligomeren oder Polymeren, die einen apolaren Polyisobutylenteil mit mindestens 40 Kohlenstoffatomen und mindestens einen endständigen polaren Teil aufweisen, der aus Carbonsäuren oder Dicarbonsäuren, ihren Anhydriden oder ihren Derivaten besteht, und ihren Gemischen ausgewählt ist, der einzige in der Zusammensetzung enthaltene Emulgator ist.

13. Verfahren zur kosmetischen Behandlung, das das Auftragen einer Zusammensetzung nach einem der Ansprüche 1 bis 12 auf die Haut umfasst.

14. Verfahren zur kosmetischen Behandlung, das dazu dient, zu beleben, zu regenerieren und/oder die Falten der Haut zu glätten und/oder die Hautalterung zu bekämpfen und/oder das Hautgewebe gegen Angriffe aus der Umwelt zu stärken, und das das Aufbringen einer Zusammensetzung nach einem der Ansprüche 1 bis 12 auf die Haut umfasst.

15. Verwendung einer Zusammensetzung nach einem der Ansprüche 1 bis 12 für die Herstellung eines dermatologischen Präparats, das dazu vorgesehen ist, die Haut, die Körperhaare und/oder die Haare zu depigmentieren und/oder die schädlichen Wirkungen der UV-Strahlung zu bekämpfen.

16. Kosmetische Verwendung einer Zusammensetzung nach einem der Ansprüche 1 bis 12, um zu beleben, zu regenerieren und/oder die Falten der Haut zu glätten und/oder die Hautalterung zu bekämpfen und/oder das Hautgewebe gegen Angriffe aus der Umwelt zu stärken.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5935584 A
- WO 9843598 A
- WO 9624325 A
- EP 0755674 A
- US 5981578 A
- US 6020367 A
- WO 0078283 A
- US 6194452 B
- US 5518517 A
- EP 1172089 A
- EP 1316303 A
- US 4234435 A
- US 4708753 A

- US 5129972 A
- US 4931110 A
- GB 2156799 A
- US 4919179 A
- JP 2295912 A
- US 5412004 A
- US 5811487 A
- EP 895779 A
- EP 524109 A
- WO 9910318 A
- WO 9932077 A
- WO 9922707 A
- EP 1038519 A

**Littérature non-brevet citée dans la description**

- *Pharm. Acta. Helv.,* 1969, vol. 44, 611-667
- *STP Pharma,* 1985, vol. 4, 281-286

- *J. Am. Acad. Dermatol.,* 1996, vol. 34, 29-33